# EUROPÄISCHE PATENTSCHRIFT

(11) **EP 3 513 709 B1**
(45) Veröffentlichungstag und Bekanntmachung des Hinweises auf die Patenterteilung: **17.07.2024**
(21) Anmeldenummer: 19152367.9
(22) Anmeldetag: 17.01.2019
(51) Int. Cl.: A61B 17/34, A61B 5/00, A61B 5/145

(54) **SYSTEM ZUR ANALYSE EINES PATIENTEN MITTELS EINES TRANSKUTANEN SENSORS**
SYSTEM FOR ANALYSING A PATIENT BY MEANS OF A TRANSCUTANEOUS SENSOR
SYSTÈME D'ANALYSE D'UN PATIENT AU MOYEN D'UN CAPTEUR TRANSCUTANÉ

(30) Priorität: 22.01.2018 DE 102018101313
(43) Veröffentlichungstag der Anmeldung: 24.07.2019
(73) Patentinhaber: EyeSense GmbH, 63762 Großostheim (DE)
(72) Erfinder: Müller, Achim, 63762 Großostheim (DE); Meissner-Braun, Tom, 88662 Überlingen (DE); Pischan, Matthias, 64287 Darmstadt (DE)
(74) Vertreter: LBP Lemcke, Brommer & Partner Patentanwälte mbB

(56) Entgegenhaltungen:
- US-A1- 2013 267 811
- US-A1- 2017 188 912
- US-A1- 2017 303 831

## Beschreibung

Die Erfindung betrifft ein System zur Analyse eines Patienten mittels eines transkutanen Sensors gemäß Oberbegriff des Anspruchs 1.

Für vielfältige medizinischen Anwendungen ist das Einführen eines Sensors in einen Patienten notwendig, insbesondere, um Messwerte des Patienten zu erhalten, beispielsweise Glukosewerte oder Laktosewerte.

Aus US 2004/0133164 A1 ist ein System zur Analyse eines Patienten mittels eines transkutanen Sensors bekannt, welches eine Basiseinheit, einen Injektor und eine Detektionseinheit umfasst. Die Basiseinheit wird an der Haut des Patienten angeklebt. Anschließend kann der Injektor auf die Basiseinheit aufgeschoben werden, um einen Sensor transkutan zu injizieren. Anschließend wird der Injektor von der Basiseinheit abgenommen und die Detektionseinheit an der Basiseinheit angeordnet, sodass mittels des Sensors Glukosewerte des Patienten durch die Detektionseinheit ermittelt werden können.

Der Sensor wird nach dem transkutanen Injizieren umgebogen und zwischen einer Basisplatte und einer Abdeckung zur Fixierung eingeklemmt.

Es sind jedoch mehr Freiheiten hinsichtlich der Geometrie und Materialbeschaffenheit des Sensors wünschenswert. Darüber hinaus können beim Umbiegen des Sensors Unannehmlichkeiten für den Patienten entstehen.

Aus US 2017/303831 A1 ist eine Sensorinjektionsanordnung bekannt, mit der ein Sensor über ein Nadelelement in einen lebenden Körper eingeführt werden kann. Dafür wird eine Sensoreinführungsvorrichtung an einer Basisplatte befestigt, welche am lebenden Körper angeordnet ist. Nach Einführung des Sensors wird dieser in einem Klemmabschnitt fixiert, um eine Bewegung des Sensors zu verhindern.

Aus US 2013/267811 A1 ist eine Vorrichtung bekannt, mit der ein Sensor in einen Patienten eingeführt werden kann. Dafür wird eine Injektionsvorrichtung am Patienten angeordnet und der Sensor über eine Einführnadel in das Gewebe des Patienten injiziert. Eine Elektronikeinheit ist dazu ausgebildet, den Sensor nach seiner Einführung in den Patienten elektrisch zu kontaktieren und zu fixieren.

Aus US 2017/0188912 A1 ist ein System zur Analyse eines Patienten mittels eines transkutanen Sensors bekannt, wobei das System eine an dem Patienten angebrachte Basiseinheit und eine Injektionsvorrichtung für einen Sensor umfasst, welcher mit einem Sensormodul gekoppelt ist, wobei das Sensormodul über Federn mit einer abnehmbaren elektronischen Einheit zur Messung und Verarbeitung der Sensordaten in elektrischer Verbindung steht.

Der vorliegenden Erfindung liegt die Aufgabe zugrunde, ein System zur Analyse eines Patienten mittels eines transkutanen Sensors zur Verfügung zu stellen, welches einen sicheren Halt des Sensors bei angeordneter Detektionseinheit bei geringen Anforderungen hinsichtlich der Geometrie und Materialbeschaffenheit des Sensors ermöglicht. Weiterhin sollen Unannehmlichkeiten bei Anordnen der Detektionseinheit vermieden werden.

Gelöst ist diese Aufgabe durch ein System zur Analyse eines Patienten mittels eines transkutanen Sensors gemäß Anspruch 1. Vorteilhafte Ausgestaltungen des erfindungsgemäßen Systems finden sich in den abhängigen Unteransprüchen.

Das erfindungsgemäße System zur Analyse eines Patienten mittels eines transkutanen Sensors weist eine Basiseinheit zum Anbringen an den Patienten, einen lösbar mit der Basiseinheit verbindbaren Injektor zum transkutanen Einführen des Sensors in die Patienten und eine lösbar mit der Basiseinheit verbindbare Detektionseinheit zum Erzeugen von Messdaten mittels des Sensors auf.

Die grundsätzliche Handhabung des erfindungsgemäßen Systems kann somit wie bekannt erfolgen, indem die Basiseinheit an dem Patienten angeordnet wird, insbesondere an der Haut des Patienten angeklebt wird. Hierbei kann der Injektor bereits während des Anbringens der Basiseinheit an den Patienten an der Basiseinheit angeordnet sein. Ebenso kann zunächst die Basiseinheit an dem Patienten angebracht werden und anschließend wird der Injektor an der Basiseinheit angeordnet. Mittels des Injektors wird der Sensor in den Patienten injiziert, insbesondere bevorzugt transkutan injiziert. Anschließend wird der Injektor von der Basiseinheit abgenommen und die Detektionseinheit an der Basiseinheit angeordnet, um Messdaten zu erzielen, insbesondere um Glukose- oder Laktosewerte des Patienten zu ermitteln, wobei die Basiseinheit eine Haltevorrichtung aufweist, welche derart zusammenwirkend mit Injektor und Detektionseinheit ausgebildet ist, dass in einer Detektionskonfiguration bei an der Basiseinheit angeordneter Detektionseinheit mittels der Haltevorrichtung ein Anpressdruck an den Sensor zum reibschlüssigen Fixieren ausgebildet ist und in einer Injektionsposition bei an der Basiseinheit angeordnetem Injektor mittels der Haltevorrichtung ein gegenüber der Detektionskonfiguration geringerer Anpressdruck an den Sensor ausgebildet ist, und wobei die Haltevorrichtung mindestens ein Hebelelement aufweist, welches derart angeordnet und ausgebildet ist, dass das Hebelelement mittels Anbringen der Detektionseinheit in Richtung des Sensors bewegbar ist.

Wesentlich ist, dass die Haltevorrichtung eine elastische Masse aufweist, welche ausgebildet ist, von dem Sensor durchdrungen zu werden, und wobei das Hebelelement mittelbar auf dem Sensor anliegt, um über die elastische Masse den Anpressdruck auf den Sensor zu übertragen

Die Basiseinheit weist somit eine Haltevorrichtung auf. Diese wird als Bestandteil der Basiseinheit mit dieser an dem Patienten angeordnet und befindet sich somit auch in der Injektionskonfiguration an dem Patienten. In der Injektionskonfiguration wird mittels der Haltevorrichtung jedoch ein gegenüber der Detektionskonfiguration geringerer Anpressdruck, insbesondere bevorzugt kein Anpressdruck, an den Sensor ausgebildet.

Die vorliegende Erfindung ermöglicht somit, durch Ausbilden des Anpressdrucks an den Sensor in Detektionskonfiguration eine Fixierung des Sensors auszubilden, welche eine sichere Messdatenübertragung zwischen Sensor und Detektionseinheit ermöglicht und darüber hinaus dem Patienten Unannehmlichkeiten erspart, da durch die Fixierung keine oder zumindest eine verringerte Bewegung des Sensors relativ zu der Basiseinheit erfolgt und auch bei Benutzung kein Anordnen eines separaten Halteelements und/oder Bearbeiten, insbesondere Umbiegen, des Sensors durch den Benutzer notwendig ist. Durch das Hebelelement wird in konstruktiv unaufwendiger Weise ein erhöhter Anpressdruck an den Sensor in Detektionskonfiguration gegenüber der Injektionskonfiguration erzielt.

Vorzugsweise weist die Haltevorrichtung ein mit dem Hebelelement zusammenwirkendes elastisches Rückstellelement auf, welches eine von dem Sensor abgewandte Rückstellkraft auf das Hebelelement erzeugend ausgebildet ist. Hierdurch wird in konstruktiv einfacher Weise eine Grundstellung des Hebelelementes ausgebildet, in welcher das Hebelelement keinen oder nur einen geringen Anpressdruck auf den Sensor ausübt. Detektionseinheit und Hebelelement sind derart zusammenwirkend ausgebildet, dass bei Anordnen der Detektionseinheit an der Basiseinheit das Hebelelement in Richtung des Sensors bewegt wird, sodass die Rückstellkraft des elastischen Rückstellelements überwunden und ein Anpressdruck auf den Sensor erzeugt wird.

Es liegt im Rahmen der Erfindung, dass die Haltevorrichtung zumindest ein Anschlagselement aufweist, welches derart angeordnet ist, dass bei Bewegen des Hebelelementes in Richtung Sensor der Sensor sich zwischen Hebelelement und Anschlagselement befindet, sodass ein Anpressdruck zwischen Anschlagselement und Hebelelement auf den Sensor ausgebildet wird. Insbesondere ist es vorteilhaft, dass das Anschlagselement ausgehend von dem Hebelelement auf der dem Hebelelement gegenüberliegenden Seite des Sensors angeordnet ist.

In einer vorteilhaften Weiterbildung weist die Haltevorrichtung mehrere Hebelelemente auf. Hierdurch kann ein größerer und/oder gleichmäßigerer Anpressdruck auf den Sensor erzielt werden. Bevorzugt weist jedes der Hebelelemente ein elastisches Rückstellelement auf. Hierbei kann ein gemeinsames elastisches Rückstellelement eine von dem Sensor abgewandte Rückstellkraft für jedes Hebelelement ausbilden. Bevorzugt ist jedem Hebelelement ein separates elastisches Rückstellelement zugeordnet.

Die mehreren Hebelelemente sind bevorzugt den Sensor umgebend angeordnet. Hierdurch kann in konstruktiv unaufwendiger Weise ein Anpressdruck zwischen den Hebelelementen ausgebildet werden, ohne dass Anschlagselemente notwendig sind.

Bevorzugt ist der Injektor ausgebildet, den Sensor in einer Injektionsrichtung entlang einer Injektionsachse in den Patienten einzuführen, insbesondere transkutan einzuführen. Die Injektionsachse steht bevorzugt senkrecht zu einer Hauptebene der Basiseinheit, welche Hauptebene parallel zu der Haut des Patienten am Ort der Injektion liegt.

Bevorzugt sind die Hebelelemente in einer gemeinsamen Ebene angeordnet, insbesondere bevorzugt in einer gemeinsamen, senkrecht zur Injektionsachse stehenden Ebene.

Die Hebelelemente sind bevorzugt auf einem Kreis, welcher den Sensor umschließt, angeordnet. Insbesondere befindet sich der Sensor bevorzugt im Mittelpunkt dieses Kreises. Bevorzugt sind die Hebelelemente gleich verteilt auf der Kreislinie angeordnet, das heißt jeweils zwei benachbarte Hebelelemente schließen mit dem Kreismittelpunkt bevorzugt einen identischen Winkel ein.

Die Hebelelemente sind somit bevorzugt ringförmig, insbesondere bevorzugt gleich verteilt den Sensor umgebend angeordnet. Die Detektionseinheit weist bevorzugt eine Anpressfläche auf, welche derart angeordnet ist, dass bei Anordnen der Detektionseinheit an der Basiseinheit ein Anpressdruck auf das Halteelement ausgebildet wird, insbesondere in Richtung des Sensors.

Hierdurch wird in konstruktiv einfacher Weise bei Anordnen der Detektionseinheit an der Basiseinheit mittels des Injektors über die Anpressfläche ein Anpressdruck auf das Halteelement und somit ein Bewegen des Halteelementes in Richtung des Sensors ausgebildet.

Das elastische Material kann im Auslieferungszustand ohne Öffnung für den Sensor ausgebildet sein und wird während des Injektionsvorgangs in diesem Fall von dem Injektor durchdrungen.

Es ist jedoch vorteilhaft, dass das elastische Material eine Durchgangsöffnung für den Sensor aufweist, welche zum reibschlüssigen Anliegen an den Sensor ausgebildet ist, insbesondere zum Presssitz.

Das elastische Material ermöglicht einerseits eine zusätzliche Fixierung des Sensors. Darüber hinaus kann in konstruktiv einfacher Weise ein Anpressdruck durch Druckausüben auf das elastische Material erzielt werden.

Insbesondere ist es vorteilhaft, wenn zumindest ein, insbesondere mehrere Hebelelemente an oder in dem elastischen Material angeordnet sind.

Zum stabilen, lösbaren Anordnen an der Basiseinheit sind Basiseinheit und Detektionseinheit und/oder Basiseinheit und Injektor zum lösbaren Verbinden mittels eines Bajonettverschlusses ausgebildet.

Die Haltevorrichtung ist bevorzugt derart mit dem Injektor zusammenwirkend ausgebildet, dass der Sensor in einer Injektionsposition die Haltevorrichtung durchdringend anordenbar ist. Hierdurch erfolgt in konstruktiv unaufwendiger Weise eine Anordnung, welche das Ausüben eines Anpressdrucks an den Sensor bei Anordnen der Detektionseinheit an der Basiseinheit ermöglicht.

Der Injektor ist bevorzugt derart zusammenwirkend mit der Basiseinheit ausgebildet, dass nach Beenden des Injektionsvorgangs der Sensor transkutan injiziert ist und sich somit teilweise im Gewebe des Patienten und teilweise außerhalb befindet. Der außerhalb des Gewebes des Patienten liegende Bereich wird bei dem anschließenden Anordnen der Detektionseinheit an der Basiseinheit mittels der Haltevorrichtung der Basiseinheit durch den erhöhten Anpressdruck fixiert.

Bevorzugt ist in Detektionskonfiguration mit Sensor in Injektionsposition (nach Abschluss des Injektionsvorgangs) die Detektionseinheit mit einem Koppelelement der Detektionseinheit an den Sensor anliegend ausgebildet.

Es liegt im Rahmen der Erfindung, dass der Sensor ein elektrischer Sensor ist und elektrische Anschlüsse aufweist. Entsprechend kann das Koppelelement der Detektionseinheit elektrische Kontakte aufweisen, welche derart ausgebildet sind, dass bei Anordnen der Detektionseinheit an der Basiseinheit die elektrischen Kontakte leitend mit korrespondierenden elektrischen Kontakten des Sensors verbunden sind.

Das erfindungsgemäße System ist insbesondere für die Verwendung eines optischen Sensors geeignet. Solche Sensoren weisen typischerweise an einem proximalen Ende einen Bereich auf, dessen optische Eigenschaft sich abhängig von den zu detektierenden Stoffen im Gewebe des Patienten ändert. Dieser proximale Bereich liegt in Injektionsposition des Sensors im Gewebe des Patienten. An einem distalen Bereich, welcher in Injektionsposition des Sensors außerhalb des Gewebes des Patienten liegt, können Informationen optisch übertragen werden. Insbesondere ist es vorteilhaft, dass der Sensor zum Ein- und Auskoppeln von Strahlung, insbesondere von Licht in einem distalen Bereich, insbesondere am distalen Ende, ausgebildet ist. Das Koppelelement ist in dieser bevorzugten Ausführungsform zum Ein- und Auskoppeln von Strahlung an dem distalen Ende des Sensors ausgebildet, insbesondere zum Ein- und Auskoppeln von Licht. Das Koppelelement ist somit bevorzugt als optisches Koppelelement ausgebildet. Die Grundprinzipien einer solchen optische Messung sind in WO2016128334A1 und WO2006092317A1 beschrieben.

Das Einführen, insbesondere transkutane Einführen des Sensors erfolgt bevorzugt mittels einer Kanüle. Hierbei liegt die Verwendung einer geschlossenen Kanüle mit einem Lumen oder einer Schlitzkanüle, welche zumindest im proximalen Bereich der Kanüle, welche in den Patienten eindringt, einen Schlitz aufweist, im Rahmen der Erfindung.

Der Injektor ist bevorzugt derart ausgebildet, dass während des Injektionsvorgangs die Kanüle mit dem Sensor transkutan in das Gewebe des Patienten eindringt und anschließend lediglich die Kanüle, nicht jedoch der Sensor, wieder aus dem Gewebe des Patienten herausgezogen wird. Bevorzugt wird hierbei der Sensor mittels eines Halteelementes des Injektors gehalten, um ein Herausziehen des Sensors zu vermeiden, insbesondere wie in DE102018101275.6 oder DE102018101283.7 beschrieben.

Die Haltevorrichtung ist daher bevorzugt in Injektionskonfiguration zum Durchdringen mittels einer Kanüle des Injektors ausgebildet. Insbesondere ist in der vorteilhaften Ausführungsform bei Vorsehen eines elastischen Materials das elastische Material dazu ausgebildet, von der Kanüle durchdrungen zu werden. Weitere bevorzugte Merkmale und Ausführungsformen werden im Folgenden anhand eines Ausführungsbeispiels und Figuren erläutert. Dabei zeigt:
- Figur 1: eine Basiseinheit des Ausführungsbeispiels in Draufsicht von oben;
- Figur 2: die Basiseinheit in Draufsicht von unten;
- Figur 3: einen Injektor und eine Detektionseinheit des Ausführungsbeispiels, welche jeweils auf einer Basiseinheit angeordnet sind, in perspektivischer Darstellung;
- Figur 4: eine Schnittdarstellung des auf der Basiseinheit angeordneten Injektors vor Injektion gemäß Schnittlinie A-A in Fig. 1;
- Figur 5: einen Detailausschnitt aus Figur 4 nach Einführen einer Kanüle des Injektors in ein Gewebe des Patienten entlang einer Schnittlinie in etwa senkrecht zu A-A in Fig.1 und
- Figur 6: eine Schnittdarstellung der auf der Basiseinheit angeordneten Detektionseinheit gemäß Schnittlinie A-A in Fig. 1.

In den Figuren bezeichnen gleiche Bezugszeichen gleiche oder gleichwirkende Elemente.

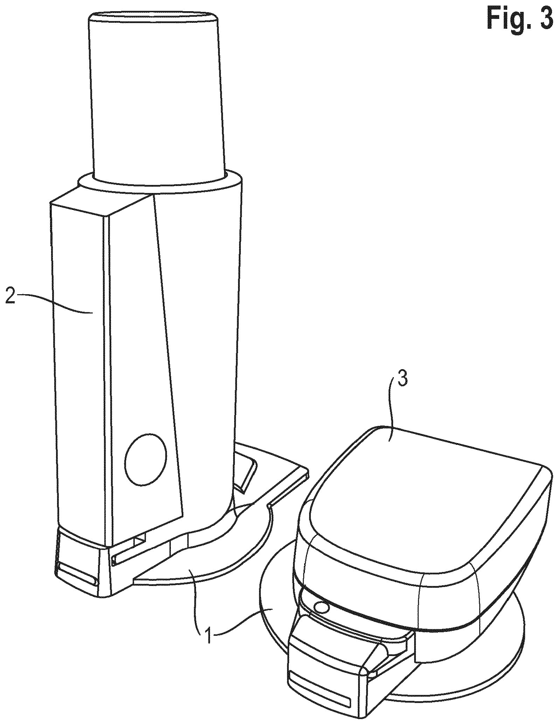

In den Figuren ist ein Ausführungsbeispiel eines erfindungsgemäßen Systems zur Analyse eines Patienten mittels eines transkutanen Sensors dargestellt. Wie insbesondere in Figur 3 ersichtlich, umfasst das System eine Basiseinheit 1 zum Anbringen an den Patienten, einen lösbar mit der Basiseinheit verbindbaren Injektor 2 zum transkutanen Einführen eines Sensors 5 in den Patienten und eine lösbar mit der Basiseinheit 1 verbindbare Detektionseinheit 3 als Detektionselement zum Erzeugen von Messdaten mittels des Sensors 5. In Figur 3 ist aus Gründen der besseren Nachvollziehbarkeit die Basiseinheit 1 zweifach dargestellt, einmal mit angeordnetem Injektor 2 und einmal mit angeordneter Detektionseinheit 3.

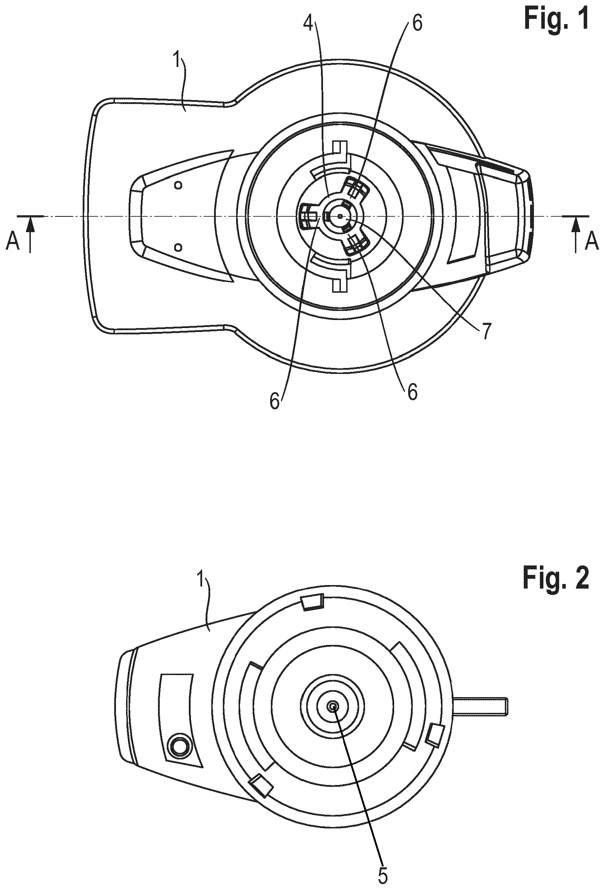

Bei Benutzung wird die Basiseinheit 1 mit der in Figur 2 in Draufsicht dargestellten Unterseite an der Haut des Patienten angeklebt. In dem vorliegenden Ausführungsbeispiel geschieht dies mit bereits an der Basiseinheit 1 angeordnetem Injektor 2. Ebenso kann zunächst die Basiseinheit an den Patienten angeordnet werden und anschließend der Injektor mit der Basiseinheit verbunden werden.

Die Basiseinheit 1 weist eine Haltevorrichtung 4 auf, welche mit Injektor 2 und Detektionseinheit 3 zusammenwirkend ausgebildet ist, sodass in einer Detektionskonfiguration bei an der Basiseinheit angeordneter Detektionseinheit mittels der Haltevorrichtung ein Anpressdruck an den Sensor 5 zum reibschlüssigen Fixieren ausgebildet ist und in einer Injektionskonfiguration bei an der Basiseinheit angeordnetem Injektor mittels der Haltevorrichtung 4 ein gegenüber der Detektionskonfiguration geringerer Anpressdruck an den Sensor 5 ausgebildet ist. Dies wird nachfolgend anhand der Figuren näher erläutert:
Wie in Figur 1 ersichtlich, ist die Haltevorrichtung 4 im Zentrum mehrerer konzentrisch angeordneter ringförmiger Elemente angeordnet. Die äußeren ringförmigen Elemente dienen zum Ausbilden eines Bajonettverschlusses bei Anordnen des Injektors 2 oder der Detektionseinheit 3 an der Basiseinheit 1. Mittig in dieser konzentrischen Anordnung befindet sich die Haltevorrichtung 4. Diese weist im vorliegenden Ausführungsbeispiel drei Hebelelemente 6 auf, welche in einer elastischen Masse 7 teilweise eingebettet sind.

Wie in Figur 1 in der Draufsicht von oben ersichtlich, umgibt die elastische Masse 7 eine mittige zylindrische Ausnehmung, welche in Draufsicht von oben gemessen Figur 1 senkrecht zur Zeichenfläche steht. Diese zylindrische Ausnehmung wird während des Injektionsvorgangs von einer Kanüle 8 des Injektors zusammen mit dem Sensor 5 durchdrungen und nach dem Injektionsvorgang durchdringt der transkutan injizierte Sensor 5 die elastische Masse 7 am Ort der zylindrischen Ausnehmungen. Auf einem Kreis, dessen Mittelpunkt der zylindrischen Ausnehmung entspricht, sind gleich verteilt die drei Hebelelemente 6 angeordnet, das heißt jeweils zwei benachbarte Hebelelemente 6 schließen mit dem Kreismittelpunkt (der zylindrischen Ausnehmung) jeweils einen gleichen Winkel ein, vorliegend 120°.

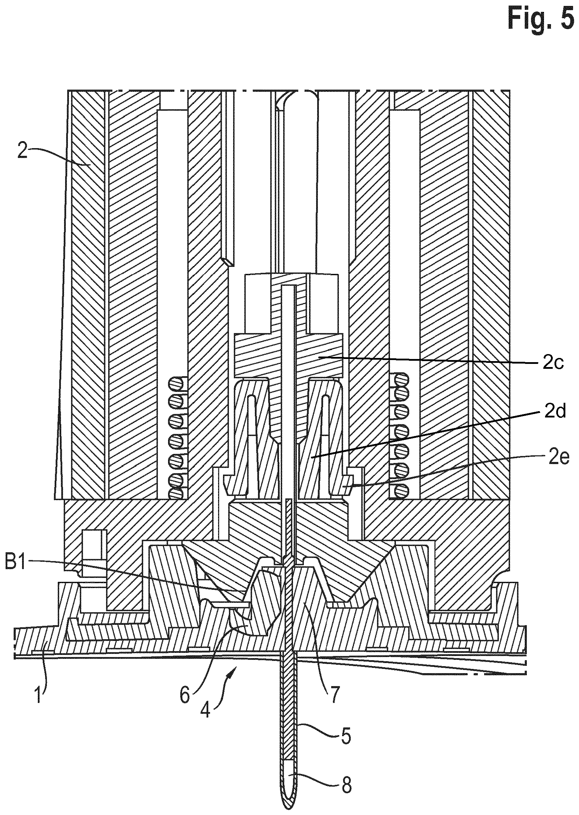

In Draufsicht von unten gemäß Figur 2 ist ersichtlich, dass im Bereich der zylindrischen Ausnehmung die Basiseinheit 1 eine kreisförmige Öffnung aufweist, sodass Kanüle 8 und Sensor 5 (welche z.B. in Figur 5 dargestellt sind), die Basiseinheit 1 an dieser Öffnung durchdringen können. In diesem Ausführungsbeispiel erfolgt die Injektion somit entlang einer Injektionsachse, welche in den Figuren 1 und 2 senkrecht zur Zeichenebene steht und am Ort der zylindrischen Ausnehmung der elastischen Masse 7 erfolgt bzw. am Ort des in Figur 2 dargestellten Sensors 5.

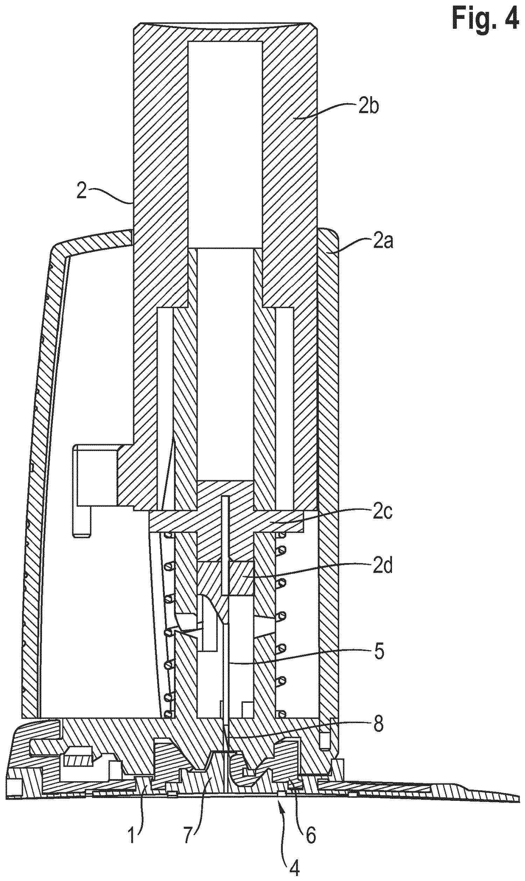

In Figur 4 ist ein Schnitt durch den an der Basiseinheit 1 angeordneten Injektor 2 dargestellt. Die Schnittebene steht senkrecht zu den Darstellungen gemäß Figur 1 und Figur 2 und ist derart gewählt, dass die Injektionsachse sowie der Sensor in der Schnittebene liegen. In Figur 1 ist die Schnittebene mit A-A gekennzeichnet.

Der Injektor 2 weist ein Basiselement 2a sowie verschiebbar an dem Basiselement angeordnete Elemente, Schiebelement 2b, Kanülenoberteil 2c und Halteelement 2d auf. Bei dem Injektionsvorgang wird das Schiebeelement 2b durch den Benutzer heruntergedrückt. Diese Bewegung wird über das Kanülenoberteil 2c auf die Kanüle 8 übertragen, welche an dem Kanülenoberteil 2c angeordnet ist. Die Kanüle 8 ist als Schlitzkanüle ausgebildet und weist in einem proximalen Bereich einen durchgehenden Schlitz auf. Unterhalb des Kanülenoberteils 2c ist das Halteelement 2d angeordnet. Dieses weist eine Nocke auf, welche durch den Schlitz in die Kanüle eingreift. Der Sensor 5 ist in der Kanüle in dem geschlitzten Bereich unterhalb der Nocke des Halteelementes 2d angeordnet. Die durch den Benutzer ausgelöste Schiebebewegung wird über das Schiebeelement 2b und das Kanülenoberteil 2c auch auf das Halteelement 2d übertragen, sodass eine Relativbewegung zwischen Sensor 5 und Kanüle 8 beim Injektionsvorgang vermieden wird, da der Sensor 5 nach oben hin in Anschlag mit der Nocke des Halteelementes 2b steht.

Durch die vorgenannte Schiebebewegung werden somit insbesondere Kanüle 8 und Sensor 5 entlang der Injektionsachse verschoben und durchdringen die elastische Masse 7 im Bereich der zylindrischen Öffnung und somit auch die Haltevorrichtung 4.

Wie in Figur 5 ersichtlich, weist der Injektor 2 im unteren Bereich B1 eine konische Fläche auf, welche in der Schnittdarstellung gemäß Figur 5 somit als schräge Fläche ersichtlich ist. Wesentlich ist, dass in diesem Bereich B1 kein Kontakt oder zumindest keine Kraftübertragung zwischen der schrägen Fläche und dem Hebelelement 6 besteht. Es wird somit bei an dem Basiselement 1 angeordnetem Injektor 2 kein Anpressdruck auf die Hebelelemente 6 ausgebildet.

In Figur 5 ist der Zustand nach transkutanem Einführen von Kanüle 8 und Sensor 5 dargestellt. Anschließend erfolgt manuell durch Herausziehen des Schiebeelementes durch den Benutzer oder durch Auslösen einer vorgespannten Injektionsfeder eine Gegenbewegung, bei welcher zumindest das Kanülenoberteil 2c entlang der Injektionsachse nach oben verschoben wird. Im heruntergedrückten Zustand rastet das Halteelement 2d mit Rastelementen 2e jedoch an dem Basiselement 2a des Injektors 2 ein, sodass das Halteelement 2d nicht nach oben verschoben wird. Hierdurch ist sichergestellt, dass bei Herausziehen der Kanüle 8 kein Herausziehen des Sensors 5 erfolgt, da die oberhalb des Sensors 5 angeordnete Nocke des Halteelements 2d, welche in den Schlitz der Kanüle 8 eingreift, ein Herausziehen des Sensors nach oben verhindert.

Der Injektor ist bevorzugt wie in DE102018101275.6 oder DE10 2018 101 283.7 beschrieben ausgebildet.

Nach Beenden des Injektionsvorgangs befindet sich die Kanüle 8 somit wieder innerhalb des Injektors 2, der Sensor 5 durchdringt jedoch die Haltevorrichtung 4 und insbesondere die elastische Masse 7 und ist transkutan in das Gewebe des Patienten injiziert. In diesem Zustand erfolgt kein Anpressdruck an den Sensor 5 mittels der Hebelelemente 6. Die zylindrische Öffnung der elastischen Masse 7 weist einen geringfügig geringeren Durchmesser als der Sensor 5 auf, sodass ein leichter Presssitz des Sensors 5 in der elastischen Masse 7 besteht.

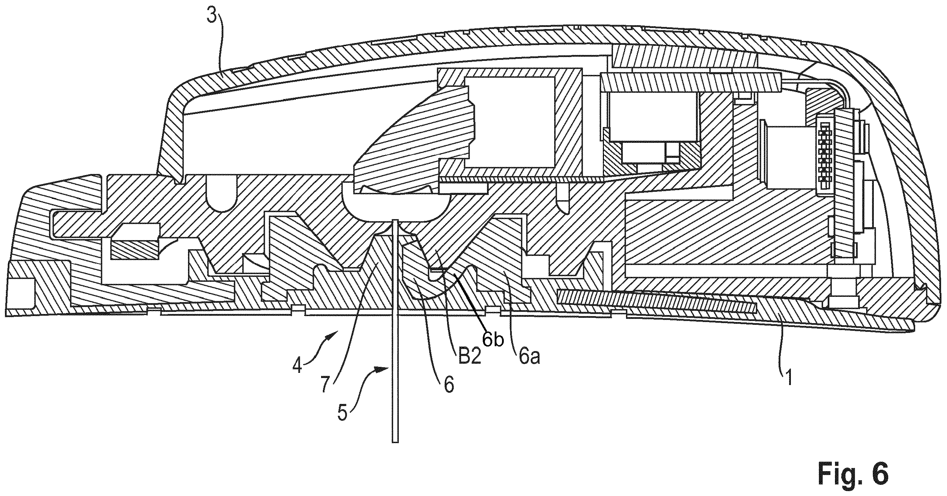

Anschließend wird der Injektor 2 von der Basiseinheit 1 gelöst und die Detektionseinheit 3 ebenfalls mittels eines Bajonettverschlusses an der Basiseinheit 1 angeordnet. Wie in Figur 6 ersichtlich, weist die Detektionseinheit 3 ebenfalls einen konisch ausgebildeten Bereich auf, welcher in der Schnittdarstellung gemäß Figur 6 insbesondere im Bereich B2 als schräge Fläche ersichtlich ist. Im Gegensatz zu dem konischen Bereich des Injektors 2 ist der konische Bereich der Detektionseinheit 3 jedoch enger ausgebildet, sodass bei an der Basiseinheit 1 angeordneter Detektionseinheit 3 ein Anpressdruck an die Hebelelemente 6 ausgebildet wird. In Figur 6 ist ersichtlich, dass das Hebelelement 6 in Richtung des Sensors 5 verschoben ist, aufgrund des Anliegens an der schrägen Fläche der Detektionseinheit 3 im Bereich B2. Das Hebelelement 6 liegt in diesem Ausführungsbeispiel zwar nicht unmittelbar an dem Sensor 5 an, dennoch wird über die elastische Masse 7 ein erheblicher Anpressdruck auf den Sensor 5 übertragen. Der geringfügige Bereich elastischer Masse 7 zwischen Sensor 5 und Hebelelement 6 ermöglicht zudem einen Druckausgleich und verhindert, dass aufgrund der harten Konsistenz des Hebelelementes 6 eine Beschädigung des Sensors 5 durch den Anpressdruck erfolgt. Ebenso können in einem alternativen Ausführungsbeispiel die Hebelelemente unmittelbar an dem Sensor 5 anliegen.

Wie insbesondere in Figur 6 ersichtlich, weist das Hebelelement 6 einen Befestigungsbereich 6a auf. Dieser ist ortsfest mit der Basiseinheit 1 verbunden. Zwischen dem Befestigungsbereich 6a und dem dem Sensor 5 zugewandten Bereich des Hebelelementes 6 ist ein elastischer Bereich ausgebildet, welcher somit als elastisches Rückstellelement 6b wirkt. Dieses Rückstellelement 6b ist derart ausgebildet, dass ohne äußeren Druck sich das Hebelelement 6 in der Grundstellung gemäß der Figuren 4 und 5 befindet und somit keinen Anpressdruck auf den Sensor 5 ausübt. Bei Anordnen der Detektionseinheit 3 wird hingegen die Rückstellkraft des elastischen Rückstellelementes 6b überwunden, um den Anpressdruck auf den Sensor 5 mittels der drei Hebelelemente 6 auszubilden.

## Patentansprüche

1. System zur Analyse eines Patienten mittels eines transkutanen Sensors, mit einer Basiseinheit (1) zum Anbringen an dem Patienten,
einem lösbar mit der Basiseinheit (1) verbindbaren Injektor (2) zum transkutanen Einführen des Sensors in den Patienten und
einer lösbar mit der Basiseinheit (1) verbindbaren Detektionseinheit (3) zum Erzeugen von Messdaten mittels des Sensors,
wobei die Basiseinheit (1) eine Haltevorrichtung (4) aufweist, welche derart zusammenwirkend mit Injektor (2) und Detektionseinheit (3) ausgebildet ist, dass in einer Detektionskonfiguration bei an der Basiseinheit (1) angeordneter Detektionseinheit (3) mittels der Haltevorrichtung (4) ein Anpressdruck an den Sensor (5) zum reibschlüssigen Fixieren ausgebildet ist und in einer Injektionskonfiguration bei an der Basiseinheit (1) angeordnetem Injektor (2) mittels der Haltevorrichtung (4) ein gegenüber der Detektionskonfiguration geringerer Anpressdruck an den Sensor (5) ausgebildet ist,
wobei die Haltevorrichtung (4) mindestens ein Hebelelement (6) aufweist, welches derart angeordnet und ausgebildet ist, dass das Hebelelement (6) mittels Anbringen der Detektionseinheit (3) in Richtung des Sensors bewegbar ist, **dadurch gekennzeichnet, dass** die Haltevorrichtung (4) eine elastische Masse (7) aufweist, welche ausgebildet ist, von dem Sensor (5) durchdrungen zu werden, und dass das Hebelelement (6) mittelbar auf dem Sensor (5) anliegt, um über die elastische Masse (7) den Anpressdruck auf den Sensor (5) zu übertragen.

2. System nach Anspruch 1,
**dadurch gekennzeichnet,**
**dass** die Haltevorrichtung (4) ein mit dem Hebelelement (6) zusammenwirkendes elastisches Rückstellelement (6b) aufweist und die elastische Masse (7) ausgebildet ist eine von dem Sensor (5) abgewandte Rückstellkraft auf das Hebelelement (6) zu erzeugen.

3. System nach einem der Ansprüche 1 oder 2,
**dadurch gekennzeichnet,**
**dass** die Haltevorrichtung (4) mehrere Hebelelemente aufweist, welches den Sensor (5) umgebend angeordnet sind.

4. System nach Anspruch 3,
**dadurch gekennzeichnet,**
**dass** die Hebelelemente ringförmig, bevorzugt gleichverteilt den Sensor (5) umgebend angeordnet sind.

5. System nach einem der vorangegangenen Ansprüche,
**dadurch gekennzeichnet,**
**dass** die Detektionseinheit (3) eine Anpressfläche aufweist, welche derart angeordnet ist, dass bei Anordnen Detektionseinheit (3) an der Basiseinheit (1) ein Anpressdruck auf ein Halteelement (2d) ausgebildet wird, insbesondere in Richtung des Sensors.

6. System nach einem der vorangegangen Ansprüche,
**dadurch gekennzeichnet,**
**dass** das elastische Material eine Durchgangsöffnung für den Sensor (5) aufweist, welche zum Reibschlüssigen Anliegen an dem Sensor (5) ausgebildet ist, insbesondere zum Presssitz.

7. System nach einem der vorangegangenen Ansprüche,
**dadurch gekennzeichnet,**
**dass** Basiseinheit (1) und Detektionseinheit (3) und/oder Basiseinheit (1) und Injektor (2) zum lösbaren Verbinden mittels eines Bajonettverschlusses ausgebildet sind.

8. System nach einem der vorangegangenen Ansprüche,
**dadurch gekennzeichnet,**
**dass** der Sensor (5) in einer Injektionsposition die Haltevorrichtung (4) durchdringend anordenbar ist.

9. System nach einem der vorangegangenen Ansprüche,
**dadurch gekennzeichnet,**
**dass** die Detektionseinheit ein Koppelelement umfasst, das in der Detektionskonfiguration am Sensor, der sich in Injektionsposition befindet, anliegend ausgebildet ist.

10. System nach Anspruch 9,
**dadurch gekennzeichnet,**
**dass** das Koppelelement als optisches Koppelelement ausgebildet ist.

11. System nach einem der vorangegangenen Ansprüche,
**dadurch gekennzeichnet,**
**dass** die Haltevorrichtung (4) in Injektionskonfiguration zum Durchdringen mittels einer Kanüle (8) des Injektors ausgebildet ist.

## Claims

1. System for analyzing a patient by means of a trans-cutaneous sensor, having a basis unit (1) for mounting on the patient,
an injector (2) which can be releasably connected to the basis unit (1) for the trans-cutaneous introduction of the sensor into the patient and
a detection unit (3) which can be releasably connected to the basis unit (1) for generating measurement data by means of the sensor,
wherein the basis unit (1) has a holding device (4) which is designed to interact with the injector (2) and detection unit (3) such that in a detection configuration, when the detection unit (3) is arranged at the basis unit (1), a pressing force at the sensor (5) is formed by means of the holding device (4) for friction-fitting fixing, and in an injection configuration, when the injector (2) is arranged at the basis unit (1) a lower pressing force at the sensor (5) than in the detection configuration is formed by means of the holding device (4),
wherein the holding apparatus (4) has at least one lever element (6) which is arranged and configured such that the lever element (6) is movable in the direction of the sensor by applying the detection unit (3), **characterised in that** the holding device (4) has an elastic mass (7) which is configured to be penetrated by the sensor (5) and that the lever element (6) rests indirectly on the sensor (5), in order to transmit the pressing force onto the sensor (5) via the elastic mass (7).

2. System according to claim 1,
**characterised in that**
the holding device (4) has an elastic returning element (6b) which interacts with the lever element (6) and the elastic mass (7) is configured to generate a returning force, facing away from the sensor (5) onto the lever element (6).

3. The system according to one of claims 1 or 2,
**characterised in that**
the holding device (4) has several lever elements which are arranged surrounding the sensor (5).

4. System according to claim 3,
**characterised in that**
the lever elements are arranged in annular fashion, preferably surrounding the sensor (5) in a regular distribution.

5. The system according to one of the preceding claims,
**characterised in that**
the detection unit (3) has a pressing surface which is arranged such that during the arrangement of the detection unit (3) at the basis unit (1) a pressing force is formed onto a holding element (2d), in particular in the direction of the sensor.

6. The system according to at least one of the preceding claims,
**characterised in that**
the elastic material has a through-opening for the sensor (5) which is configured for resting in friction-fitting manner against the sensor (5), in particular for a press-fit.

7. The system according to one of the preceding claims,
**characterised in that**
basis unit (1) and detection unit (3) and/or basis unit (1) and injector (2) are configured for releasable connection by means of a bayonet closure.

8. The system according to one of the preceding claims,
**characterised in that**
the sensor (5) can be arranged in an injection position penetrating the holding device (4).

9. The system according to one of the preceding claims,
**characterised in that**
the detection unit includes a coupling element which, in the detection configuration, is designed to rest on the sensor which is in injection position.

10. System according to claim 9,
**characterised in that**
the coupling element is designed as an optical coupling element.

11. The system according to one of the preceding claims,
**characterised in that**
the holding device (4), in injection configuration, is designed for penetration by means of a cannula (8) of the injector.

## Revendications

1. Système destiné à l'analyse d'un patient au moyen d'un capteur transcutané, avec une unité de base (1) destinée à être installée sur le patient,
un injecteur (2) pouvant être relié de manière amovible à l'unité de base (1) destiné à l'introduction transcutanée du capteur à l'intérieur du patient et
une unité de détection (3) pouvant être reliée de manière amovible à l'unité de base (1) destinée à la génération de données de mesure au moyen du capteur,
dans lequel l'unité de base (1) présente un dispositif de retenue (4) qui est réalisé pour interagir avec l'injecteur (2) et l'unité de détection (3) de telle sorte que dans une configuration de détection, lorsque l'une unité de détection (3) est disposée au niveau de l'unité de base (1), une pression d'appui est formée au niveau du capteur (5) au moyen du dispositif de retenue (4) pour une fixation par friction et dans une configuration d'injection, lorsqu'un injecteur (2) est disposé au niveau de l'unité de base (1), une pression d'appui plus faible par rapport à la configuration de détection est formée au niveau du capteur (5) au moyen du dispositif de retenue (4),
dans lequel le dispositif de retenue (4) présente au moins un élément de levier (6) qui est disposé et réalisé de telle sorte que l'élément de levier (6) est mobile en direction du capteur en appliquant l'installation de l'unité de détection (3), **caractérisé en ce que** le dispositif de retenue (4) présente une masse élastique (7) qui est réalisée pour être traversée par le capteur (5) et **en ce que** l'élément de levier (6) repose indirectement sur le capteur (5) pour transférer la pression d'appui au capteur (5) par le biais de la masse élastique (7).

2. Système selon la revendication 1,
**caractérisé en ce que**
le dispositif de retenue (4) présente un élément de rappel élastique (6b) interagissant avec l'élément de levier (6) et la masse élastique (7) est réalisée pour générer sur l'élément de levier (6) une force de rappel opposée au capteur (5).

3. Système selon l'une quelconque des revendications 1 ou 2,
**caractérisé en ce que**
le dispositif de retenue (4) présente plusieurs éléments de levier qui sont disposés autour du capteur (5).

4. Système selon la revendication 3,
**caractérisé en ce que**
les éléments de levier sont disposés de manière annulaire, de préférence répartis uniformément autour du capteur (5).

5. Système selon l'une quelconque des revendications précédentes,
**caractérisé en ce que**
l'unité de détection (3) présente une surface d'appui qui est disposée de telle sorte que, lors de l'agencement de l'unité de détection (3) au niveau de l'unité de base (1), une pression d'appui est formée sur un élément de retenue (2d), en particulier en direction du capteur.

6. Système selon l'une quelconque des revendications précédentes,
**caractérisé en ce que**
le matériau élastique présente une ouverture traversante pour le capteur (5) qui est réalisée pour reposer par friction sur le capteur (5), en particulier pour un ajustement serré.

7. Système selon l'une quelconque des revendications précédentes,
**caractérisé en ce que**
l'unité de base (1) et l'unité de détection (3) et/ou l'unité de base (1) et l'injecteur (2) sont réalisés pour une liaison amovible au moyen d'un verrouillage par baïonnette.

8. Système selon l'une quelconque des revendications précédentes,
**caractérisé en ce que**
le capteur (5) peut être disposé de manière à pénétrer dans le dispositif de retenue (4) dans une position d'injection.

9. Système selon l'une quelconque des revendications précédentes,
**caractérisé en ce que**
l'unité de détection comprend un élément de couplage qui est réalisé de manière contiguë au capteur qui se trouve en position d'injection dans la configuration de détection.

10. Système selon la revendication 9,
**caractérisé en ce que**
l'élément de couplage est réalisé en tant qu'élément de couplage optique.

11. Système selon l'une quelconque des revendications précédentes,
**caractérisé en ce que**
le dispositif de retenue (4) est réalisé dans la configuration d'injection pour pénétrer dans l'injecteur au moyen d'une canule (8).
